# EUROPEAN PATENT APPLICATION

(11) **EP 3 417 877 A1**
(43) Date of publication of application: **26.12.2018**
(21) Application number: 17753337.9
(22) Date of filing: 17.02.2017
(51) Int. Cl.: A61K 39/395, A61K 31/7105, A61K 48/00, A61P 35/00, A61P 35/04

(54) **COMPLEX CAPABLE OF INHIBITING GENETIC FUNCTION IN EXOSOME, AND CANCER PROLIFERATION AND/OR METASTASIS SUPPRESSOR**

(30) Priority: 18.02.2016 JP 2016028924
(71) Applicant: Kyoto University, Kyoto-shi, Kyoto 606-8501 (JP); Kyoto Pharmaceutical University, Kyoto-shi, Kyoto 607-8414 (JP)
(72) Inventor: YAMAYOSHI, Asako, Kyoto-shi Kyoto 606-8501 (JP); MURAKAMI, Akira, Kyoto-shi Kyoto 606-8585 (JP); ASHIHARA, Eishi, Kyoto-shi Kyoto 607-8414 (JP); KOBORI, Akio, Kyoto-shi Kyoto 606-8585 (JP)
(74) Representative: Hoffmann Eitle
(86) International application number: PCT/JP2017/005994
(87) International publication number: WO 2017/142083

(57) **Abstract**

The present invention provides a conjugate comprising an antibody or antibody fragment targeting an exosome surface antigen, and an inhibitor of a gene or an expression product thereof, wherein the antibody or antibody fragment and the inhibitor of a gene or an expression product thereof are covalently bonded either directly or via a linker, or are non-covalently bonded.

## Description

### Technical Field

The present invention relates to a conjugate capable of inhibiting the genetic function of exosome, and a cancer proliferation and/or metastasis inhibitor.

### Background Art

Recent studies found a mechanism in which various cells including T-cells, platelets, epithelial cells, immune cells, or cancer cells, release vesicles named exosome having a diameter of 40 to 100 nm, thereby transmitting information to distant cells.

In particular, a new mechanism in which the cancer cell proliferation and metastatic ability is dominated by an expression product of a gene, such as miRNA, contained in the exosome secreted in blood has been suggested and has been attracting significant attention.

To inhibit the function of miRNA in the exosome, a method using a modified nucleic acid (antisense nucleic acid) having a sequence complementary to the miRNA is generally used (Non-Patent Documents 1 to 3). However, since miRNA in the blood is encapsulated in an exosome, direct targeting of miRNA is difficult even by administering an antisense nucleic acid into the blood.

Many methods using an exosome as a drug delivery system have been known (Non-Patent Documents 3 to 5, and Patent Documents 1 and 2).

### Citation List

### Patent Documents

Patent Document 1: JP2010-285426A
Patent Document 2: JP2014-185090A

### Non-Patent Documents

Non-Patent Document 1: G. Hutvagner, M.J. Simard, C.C. Mello and P.D. Zamore, PLoS Biol., 2004, 2, E98.
Non-Patent Document 2: U.A. Orom, S. Kauppinen and A.H. Lund, Gene, 2006, 372, 137-141
Non-Patent Document 3: S. Davis, B. Lollo, S, Freier and C. Esau, Nucleic Acids Res., 2006, 34, 2294-2304
Non-Patent Document 4: Lai CP, Mardini 0, Ericsson M, Prabhakar S, Maguire CA, Chen JW, et al. Acs Nano. 2014; 8(1): 483-94
Non-Patent Document 5: Smyth T, Petrova K, Payton NM, Persaud I, Redzic JS, Graner MW, et al. Bioconjugate Chem. 2014; 25(10): 1777-84

### Summary of Invention

### Technical Problem

A major object of the present invention is to provide a novel method for inhibiting the function of a gene, such as miRNA, or an expression product thereof, contained in an exosome.

### Technical Problem

The present invention provides the following conjugate and/or cancer proliferation and/or metastasis inhibitor.
Item 1. A conjugate comprising an antibody or antibody fragment targeting an exosome surface antigen, and an inhibitor of a gene or an expression product thereof, wherein the antibody or antibody fragment and the inhibitor of a gene or an expression product thereof are covalently bonded either directly or via a linker, or are non-covalently bonded.
Item 2. The conjugate according to Item 1, wherein the exosome surface antigen is CD9, CD63, CD81 or CD147.
Item 3. The conjugate according to Item 1 or 2, wherein the antibody or antibody fragment is modified with a peptide containing at least one amino acid selected from the group consisting of cysteine, arginine, lysine and ornithine, and the inhibitor of a gene or an expression product thereof is bonded to the peptide via a covalent bond (S-S bond), a coordinate bond, or a non-covalent bond.
Item 4. The conjugate according to Item 3, wherein the peptide further comprises glycine or alanine.
Item 5. The conjugate according to Item 3, wherein the peptide is polylysine or polyarginine.
Item 6. The conjugate according to Item 5, wherein the peptide is polyarginine.
Item 7. The conjugate according to any one of Items 1 to 6, wherein the inhibitor of a gene or an expression product thereof is anti-miRNA nucleic acid, and the anti-miRNA nucleic acid is a nucleic acid that inhibits miRNA function by forming a complementary strand with miRNA contained in the exosome. Item 8. The conjugate according to Item 1, wherein the antibody or antibody fragment is anti-CD63 antibody.
Item 9. The conjugate according to Item 1, wherein the inhibitor of a gene or an expression product thereof is an inhibitor of miRNA or a gene contained in the exosome.
Item 10. The conjugate according to Item 1, wherein the inhibitor of a gene or an expression product thereof is a miRNA inhibitor. Item 11. The conjugate according to Item 1, wherein the antibody or antibody fragment is a monoclonal antibody, a single-chain antibody, Fab, Fab', F(ab')₂, Fv, or scFv.
Item 12. A cancer proliferation and/or metastasis inhibitor, comprising the conjugate according to any one of Items 1 to 11.

### Advantageous Effects of Invention

The present invention is capable of effectively inhibiting, in particular, a function of a gene contained in an exosome involved in cancer metastasis and proliferation.

### Brief Description of Drawings

Fig. 1: A schematic diagram of an action mechanism of a conjugate of the present invention.
Fig. 2: A schematic diagram showing a production scheme of a fluorescent-labeled antibody of Example 1.
Fig. 3: A confocal laser microscope image showing the results of Example 1.
Fig. 4: A schematic diagram showing a production scheme of a conjugate of Example 2.
Fig. 5: A confocal laser microscope image showing the results of Example 2 and Comparative Example 1. The upper row titled "anti-CD63 IgG + RNA(Cy5)" shows the results of Comparative Example 1, and the lower row titled "anti-CD63 IgG-9r + RNA(Cy5)" shows the results of Example 1. Phalloidin is an oligopeptide that specifically binds to a polymerized actin (F-actin) constituting the cytoskeleton, and was used for the staining of the cytoskeleton.
Fig. 6: A schematic diagram showing a production scheme of Example 3.
Fig. 7: microRNA function inhibiting effects of an anti-CD63 antibody/anti-miR conjugate was incorporated into a cell.
Fig. 8: Exosome miR21-dependent cell proliferation inhibition.
Fig. 9: Effects of anti-CD63 antibody/anti-miR nucleic acid conjugate in vivo.

### Description of Embodiments

The inhibitor of a gene or an expression product thereof is an inhibitor of the function of a gene contained in an exosome. Examples thereof include low-molecular compounds, miRNA inhibitors, DNA inhibitors, mRNA inhibitors, tRNA inhibitors, rRNA inhibitors, piRNA inhibitors, non-coding RNA inhibitors, aptamers, antibodies, F(ab')₂ fragments, single-chain antibody fragments, Fv fragments, single-chain Fv fragments, Affibody, Nanobody, and selective antibody scaffolds (e.g., diabody).

In a preferred embodiment of the present invention, examples of an inhibitor of a gene or an expression product thereof include low-molecular compounds, and anti-miRNA nucleic acids. Examples of a low-molecular compound include cisplatin, 5FU (5-fluorouracil), doxorubicin, actinomycin, mitomycin, cyclophosphamide, melphalan, and the like.

Examples of an antibody or antibody fragment include monoclonal antibodies, single-chain antibodies, Fab, Fab', F(ab')₂, Fv, and scFv.

miRNA (microRNA) is RNA contained in an exosome and having about 15 to 30 bases, in particular about 18 to 25 bases. In the present invention, "exosome" broadly encompasses vesicles released from mammalian cells. Examples of animals include humans, monkeys, cows, sheep, goats, horses, pigs, rabbits, dogs, cats, rats, mice, guinea pigs, and the like, and particularly preferably humans. Examples of mammalian cells include, in particular, tumor cells, dendritic cells, macrophage, T-cells, B cells, platelets, reticulocytes, epithelial cells, fibroblasts, and the like, and in particular, tumor cells. The diameter of the exosome is about 30 to 200 nm, preferably about 30 to 100 nm.

Examples of exosome surface antigen as the target of the antibody or antibody fragment include CD9, CD63, CD81, and CD147. Examples of preferable exosome surface antigen as the target of the antibody or antibody fragment include CD9 and CD63, and more preferably CD63.

The conjugate of the present invention comprises, as an essential component, an antibody or antibody fragment targeting an exosome surface antigen, and an inhibitor of a gene or an expression product thereof.

Examples of the antibody or antibody fragment targeting an exosome surface antigen include anti-CD9 antibody, anti-CD63 antibody, anti-CD81 antibody, anti-CD147 antibody, and fragments of these antibodies. Preferable examples include anti-CD9 antibody, anti-CD63 antibody, and antibody fragments thereof. Further preferable examples include anti-CD63 antibody, and antibody fragments thereof.

By having a sequence complementary to miRNA contained in the exosome, the anti-miRNA nucleic acid forms a complementary strand with the miRNA, thereby inhibiting the function of miRNA. The anti-miRNA nucleic acid may consist only of a sequence complementary to miRNA, or any sequence may be added at the 5' end or the 3' end of the sequence complementary to miRNA. The number of bases in the sequence to be added is 50 or less, preferably 40 or less, more preferably 20 or less, further preferably 10 or less, and particularly preferably 5 or less. The most preferable anti-miRNA nucleic acid consists only of a strand complementary to the target miRNA. The anti miRNA nucleic acid is DNA, RNA, or a nucleic acid derivative, and is preferably RNA. The nucleic acid derivative designates a derivative in which an atom (e.g., hydrogen atom, oxygen atom) of, for example, a base moiety, a ribose moiety, a phosphodiester bond moiety, or a functional group (e.g., hydroxy group, amino group) of the nucleic acid is replaced with another atom (e.g., hydrogen atom, sulfur atom), a functional group (e.g., amino group) or a C1-6 alkyl group, or is protected by a protecting group (e.g., methyl group, or acyl group), or those in which these moieties are replaced with non-natural components (e.g., a peptide bond). Examples of such nucleic acid derivatives include peptide nucleic acids (PNA) in which the base moiety is linked via a peptide bond, glycol nucleic acid (GNA), threose nucleic acid (TNA), bridged nucleic acid (BNA), a nucleic acid in which the hydrogen atom of the amino group of the base is substituted with a C₁₋₆ alkyl group, a nucleic acid with modified steric configuration of the hydroxy group in the ribose moiety, a nucleic acid having phosphorothioate in which the oxygen atom in the phosphodiester bond moiety is replaced with a sulfur atom, and the like.

The anti miRNA nucleic acid may contain one sequence complementary to the target miRNA, or two or more (e.g., 2 to 10, preferably 2, 3, 4, or 5) sequences complementary to the target miRNA either directly or via an appropriate base. Further, as the complementary sequence, a plurality of one type of sequence, or a plurality of various kinds of complementary sequences may be included. Anti miRNA nucleic acid may be a single-stranded nucleic acid, or a double-stranded nucleic acid. Examples of double-stranded nucleic acid include dsRNA and siRNA. RNA having a hairpin structure such as shRNA is also included in anti-miRNA nucleic acid. The double-stranded nucleic acid includes DNA-RNA hybrid.

The gene or an expression product thereof of an exosome to be inhibited in function is a gene involved, in particular, in cancer proliferation and metastasis.

The inhibitor of a gene or an expression product thereof, for example, an miRNA inhibitor, and the antibody or antibody fragment may be covalently bonded either directly or via a linker, or may be non-covalently bonded. Examples of a non-covalent bond include ionic bond, coordinate bond, hydrophobic interaction and the like. For example, when the antibody or antibody fragment is modified with a peptide having at least one cysteine residue, the inhibitor of a gene or an expression product thereof having an SH group may be covalently bonded via the SH group of cysteine by an S-S bond, or may be bonded by an - S-(metal ion)-S- coordinate bond via a metal ion. The number of cysteine may be one, or two or more. When the antibody or antibody fragment is modified with a peptide having at least one arginine residue, the inhibitor of an anionic gene, such as an anti-miRNA nucleic acid or an expression product thereof, may be non-covalently bonded to a cation of the arginine residue by an ionic bond. Further, if the antibody or antibody fragment is modified with a peptide having a lysine residue or an ornithine residue, an anionic gene, such as an anti-miRNA nucleic acid or an expression product thereof, may be non-covalently bonded to a cation of the lysine residue or the ornithine residue by an ionic bond, or may also be covalently bonded via the terminal amino group (NH₂) of the lysine residue or the ornithine residue either directly or via an appropriate linker.

The peptide bonded to the antibody or antibody fragment either directly or via a linker is preferably a peptide constituted of a basic amino acid selected from lysine (Lys, K), arginine (Arg, R), and ornithine (Orn). The peptide may include a glycine or alanine residue for the adjustment of linker length. The basic amino acid is more preferably lysine (Lys, K), or arginine (Arg, R). The peptide in a preferred embodiment is polyarginine or polylysine. The number of amino acids in the peptide is not particularly limited insofar as the bond of the inhibitor of an anionic gene or an expression product thereof (e.g., miRNA inhibitor) is possible; the number is, for example, 4 to 50, preferably 5 to 40, more preferably 6 to 30, further preferably 7 to 25, and particularly preferably 8 to 20. For example, when the peptide is constituted only of a basic amino acid, the number of the inhibitors of an anionic gene or an expression product thereof (e.g., miRNA inhibitor) to be bonded to the peptide is 1 or 2; preferably, the peptide and the inhibitor of an anionic gene or an expression product thereof is bonded 1:1. The number of peptides to be bonded to a single antibody or antibody fragment is 1 to 10, 1 to 8, 1 to 6, 1 to 4, or 1 to 2. By bonding a plurality of peptides to an antibody, it is possible to obtain a conjugate comprising a plurality of miRNA inhibitors. The miRNA in exosome is said to be more than 200 kinds, and when a large number of target miRNA are present, it is possible to bond a plurality of peptides to a single antibody, thereby bonding many kinds of miRNA inhibitors. Further, when a large number of surface antigens, such as CD9, CD63, CD81, or CD147, are present in the exosome, the number of antibodies may be increased.

In this specification, "antibody or antibody fragment modified with a peptide" means that a peptide is covalently bonded to an antibody or antibody fragment. Examples of the site to which the peptide is bonded include constant regions (C_{H}1, C_{H}2, C_{H}3) of the antibody or antibody fragment and constant regions such as an Fc region. The bond of the peptide to the antibody or antibody fragment may be performed according to a standard method, for example, according to Scheme 1 shown below. 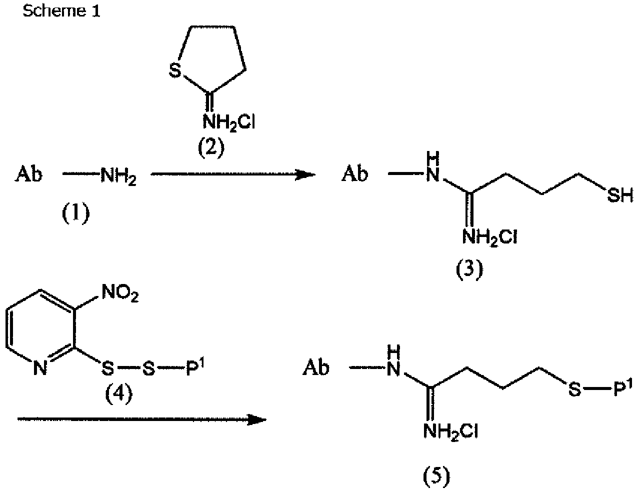 wherein Ab is an antibody or antibody fragment; NH₂ bonded to Ab is an amino group of an amino acid in a region insignificantly affecting the bond of Ab (e.g., a constant region such as CH1, CH2, CH3, or Fc region); P¹ is a peptide containing at least one amino acid selected from the group consisting of cysteine, arginine, lysine and ornithine, or an inhibitor of a gene or an expression product thereof.

The antibody or antibody fragment (1) is reacted with compound (2), thereby obtaining amidine compound (3), and amidine compound (3) is reacted with compound (4), thereby obtaining an antibody or antibody fragment (5) in which an inhibitor of a gene or an expression product thereof or a peptide is bonded. The reaction conditions in obtaining an antibody or antibody fragment (5) in which an inhibitor of a gene or an expression product thereof or a peptide is bonded may be easily determined by a person skilled in the art by referring to the disclosures of ACS Chem. Biol. 2011, 6, 962-970, or the like. Scheme 1 shown above is only an example, and any state in which an inhibitor of a gene or an expression product thereof or a peptide is covalently bonded to an antibody or antibody fragment either directly or via a linker is included in the present invention.

Examples of a linker for the bond of an inhibitor of a gene or an expression product thereof, a peptide, and an antibody or antibody fragment include -0-, -CO-, -CONH-, -NHCO-, -NH₂-, - (OCH₂CH₂)n-, a bivalent linker having a maleimide group and a succinimide group at the terminus, and the like.

The antibody or antibody fragment in which a peptide is bonded is mixed with anti-miRNA nucleic acid in water or a like solvent, thereby forming a conjugate.

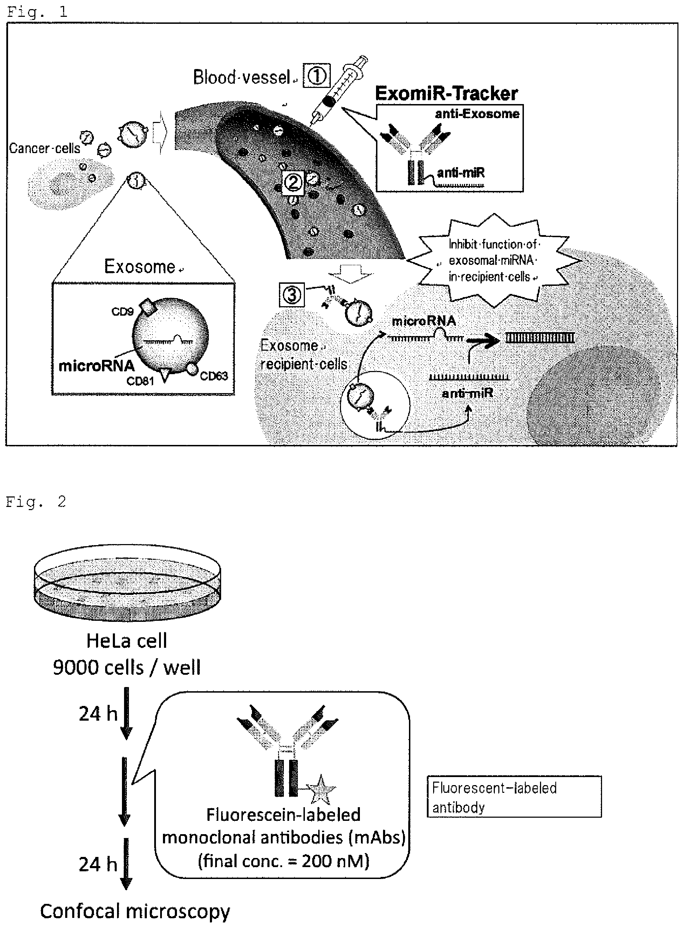

As shown in Fig. 1, when the conjugate of the present invention is injected intravenously, an antibody or antibody fragment site of the conjugate binds to an exosome. At this time, the inhibitor of a gene or an expression product thereof (anti-miR in Fig. 1) is present outside the exosome. However, when the exosome is incorporated into a cell, the inhibitor is also incorporated into a cell together with the exosome. When the exosome is broken inside the cell and the inhibitor of a gene or an expression product thereof (anti-miRNA nucleic acid in Fig. 1) is released, the function of the gene or an expression product thereof is inhibited. When the gene or an expression product thereof to be inhibited in function is involved in cancer proliferation or metastasis, the conjugate of the present invention serves as a cancer proliferation and/or metastasis inhibitor. The conjugate of the present invention may be administered in a dose of about 1 µg to 1 g for an adult per day for the inhibition of cancer proliferation and/or metastasis.

### Examples

The present invention is more specifically explained below in reference to Examples. The present invention is, however, not limited to those Examples.

In the Examples, an anti-CD63 antibody manufactured by Cosmo Bio Co., Ltd., an anti-CD9 antibody and anti-CD81 antibody manufactured by abcam, and an anti-TSG101 antibody manufactured by abnova were used.

### Example 1: Capture of fluorescent-labeled antibody into cell (Fig. 2)

It was verified whether an antibody recognizing an exosome surface antigen was incorporated into a cell together with the exosome.

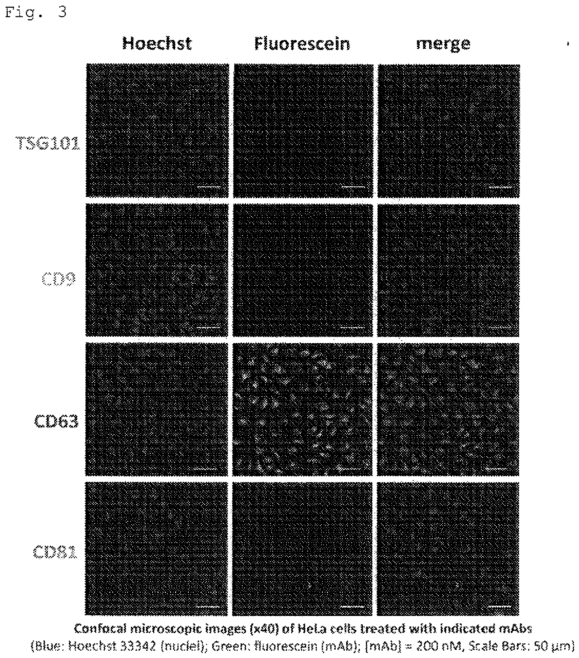

Hela cells (cervical cancer cells) were plated onto a multiwell glass-bottom dish (Matsunami Glass Ind.,Ltd) in an amount of 9000/well, and incubated at 37°C for 24 hours using a 5% CO₂ incubator. A fluorescent-labeled antibody (anti-CD63 antibody, anti-CD9 antibody, anti-CD81 antibody, and anti-TSG101 antibody) was added to each well, followed by incubation for 24 hours at 37°C using a 5% CO₂ incubator. The supernatant was removed and the cells were washed with 1×PBS. 100 µL of 4% paraformaldehyde was added, followed by incubation for 5 minutes at room temperature, thereby immobilizing the cells. The cells were washed twice with 1×PBS. 200 µL of Hoechst33342 in 1×PBS was added (final concentration = 5 µM), followed by incubation for 10 minutes at room temperature, thereby staining viable cells. The cells were washed twice with 1×PBS, and confocal laser microscope imaging was performed. Fig. 3 shows the results. As shown in Fig. 3, in the cells treated with anti-CD63 antibody (CD63), the fluorescence of fluorescein as an antibody labeling group was clearly observed. The fluorescence of fluorescein was slightly observed with respect to anti-CD9 antibody (CD9), anti-CD81 antibody (CD81). The fluorescence of fluorescein was not observed when anti-TSG101 antibody (TSG101) was used.

Although anti-CD63 antibody was incorporated in the above example using Hela cells, when Cal27 cells were used instead of Hela cells, anti-CD9 antibody was preferentially incorporated.

It was suggested that anti-CD63 antibody and anti-CD9 antibody were first bonded to an exosome present in the cell culture supernatant, and then incorporated into a cell.

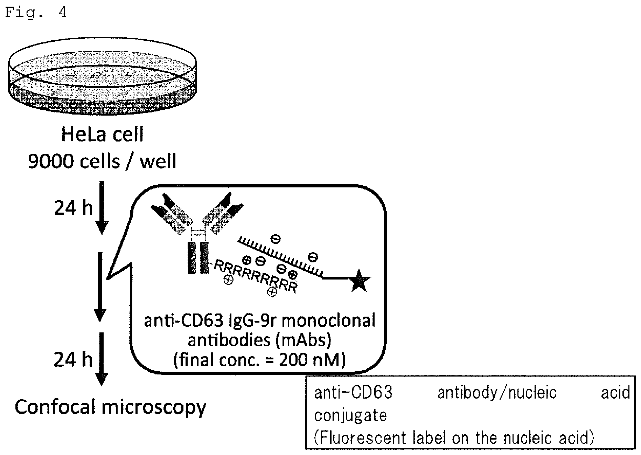

### Example 2 and Comparative Example 1: Capture of antibody/nucleic acid conjugate into cell (Fig. 4)

It was verified whether an antibody recognizing an exosome surface antigen was incorporated into a cell together with the exosome.

Hela cells (cervical cancer cells) were plated onto a multiwell glass-bottom dish (Matsunami Glass Ind., Ltd) in an amount of 9000/well, and incubated at 37°C for 24 hours using a 5% CO₂ incubator. An anti-CD63 antibody-9r/nucleic acid conjugate (anti-CD63 IgG-9r + anti-miR(Cy5)) was added to each well. The anti-miR(Cy5) used herein was 5'-Cy5-aguca auagg gugug ugaga gacuu acug- 3' (FASMAC, SEQ ID NO: 1).

As Comparative Example 1, anti-CD63 IgG and anti-miR(Cy5) were added instead of the anti-CD63 IgG-9r/nucleic acid conjugate.

Phalloidin was used for the staining of the cytoskeleton. Phalloidin is an oligopeptide that specifically binds to a polymerized actin (F-actin) constituting the cytoskeleton.

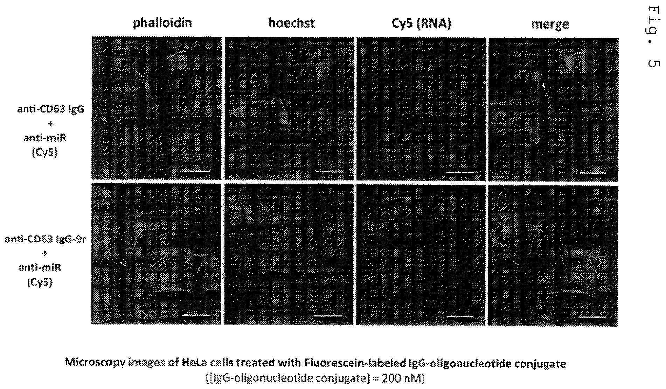

The incubation was performed at 37°C for 24 hours using a 5% CO₂ incubator. The supernatant was removed and the cells were washed with 1×PBS. 100 µL of 4% paraformaldehyde was added, followed by incubation for 5 minutes at room temperature, thereby immobilizing the cells. The cells were washed twice with 1×PBS, and 200 µL of Alexa488-labelled phalloidine solution (final concentration = 100 nM) in 1×PBS was added, followed by incubation at room temperature for 20 minutes. The phalloidine solution was removed, and 200 µL of Hoechst33342 (final concentration = 5 µM) in 1×PBS was added, followed by incubation at room temperature for 10 minutes, thereby staining viable cells. The cells were washed twice with 1×PBS, and confocal laser microscope imaging was performed. Fig. 5 shows the results. As shown in Fig. 5, it was shown that the anti-CD63 antibody/nucleic acid conjugate (anti-CD63 IgG-9r + anti-miR (Cy5)) was incorporated into a cell.

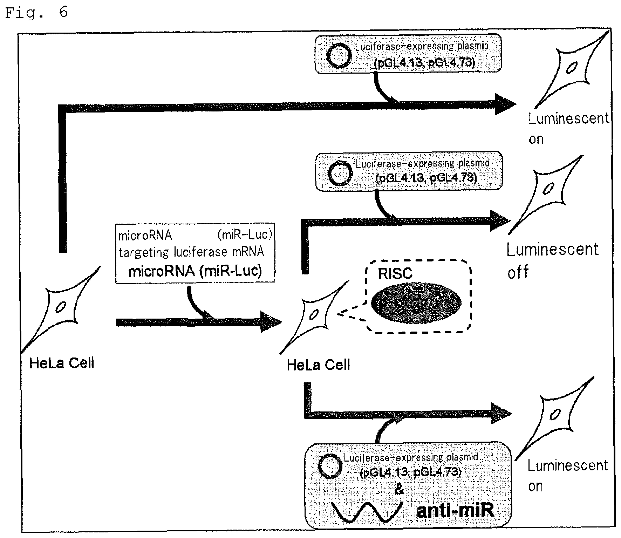

### Example 3: microRNA function inhibiting effect of anti-CD63 antibody/anti-miR nucleic acid conjugate (Fig. 6)

The exertion of microRNA function inhibiting effect after the anti-CD63 antibody/anti-miR nucleic acid conjugate was incorporated into a cell was evaluated.

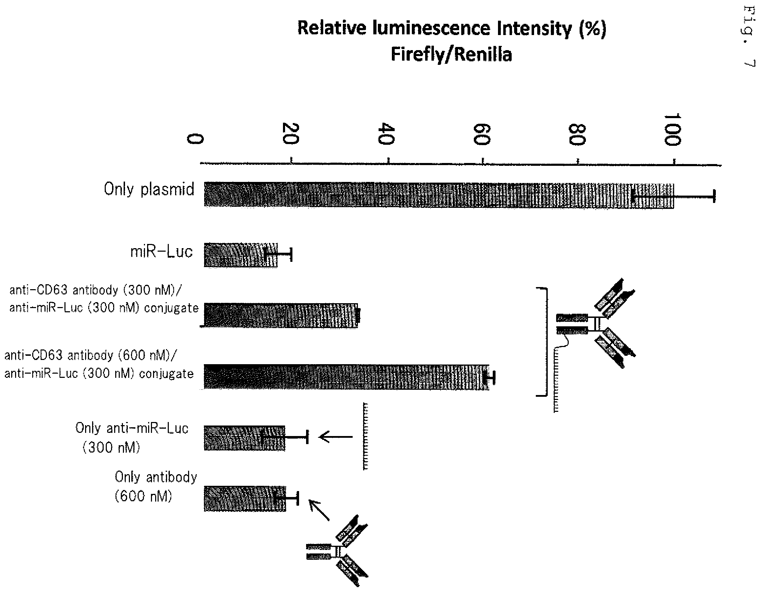

Hela cells (cervical cancer cells) were plated onto a 96-well plate in an amount of 4500/well, and incubated at 37°C for 24 hours using a 5% CO₂ incubator. microRNA(miR-Luc) targeting luciferase mRNA was introduced into each well (Lipofectamine RNAiMAX). The incubation was performed at 37°C for 18 hours using a 5% CO₂ incubator, and a luciferase-expressing plasmid (pGL4.13&pGL4.73) was introduced (Lipofectamine 2000). The incubation was performed at 37°C for 6 hours using a 5% CO₂ incubator, and either anti-CD63 antibody/anti-miR nucleic acid conjugate (anti-CD63 IgG-9r + anti-miR-Luc), only anti-miR-Luc (300 nM), or only anti-CD63 antibody (600 nM) was added and incubation was performed at 37°C for 24 hours using a 5% CO₂ incubator; then firefly luciferin was added and luciferase assay was performed. The conjugate was anti-CD63 antibody (300 nM)/anti-miR nucleic acid (300 nM), or anti-CD63 antibody (600 nM)/anti-miR nucleic acid (300 nM). Fig. 7 shows the results.

It was confirmed that the anti-CD63 antibody/anti-miR nucleic acid conjugate exerted microRNA function inhibiting effect after the conjugate was incorporated into a cell.

### Example 4: Exosome-encapsulated microRNA function inhibiting effect of anti-CD63 antibody/anti-miR nucleic acid conjugate

It was evaluated whether the anti-CD63 antibody/anti-miR nucleic acid conjugate exerted an exosome-encapsulated microRNA function inhibiting effect.

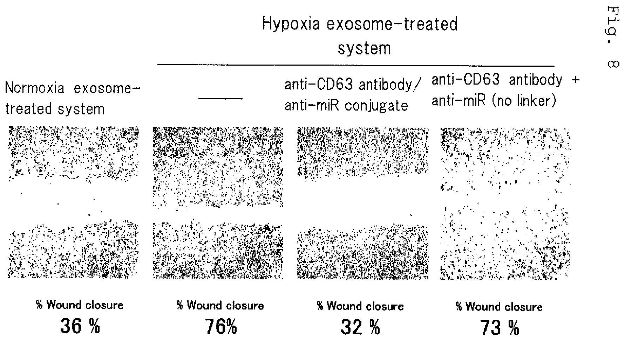

Cal27 (oral epithelial cancer cells) were plated onto a 96-well plate in an amount of 50000/well, and incubated at 37°C for 24 hours using a 5% CO₂ incubator. The cells were scratched and then cultured under hypoxia (0.1% O₂) or normoxia (20% O₂); thereafter, exosome (10 µg/ml) was added. Subsequently, in a hypoxia exosome-treated system, incubation was performed with anti-CD63 antibody/anti-miR nucleic acid conjugate (anti-CD63 IgG-9r + anti-miR21), anti-CD63 antibody + anti-miR-21 (no linker), or a no-addition system (control) at 37°C for 24 hours using a 5% CO₂ incubator. Thereafter, scratch wound closure (% Wound closure) was observed. Fig. 8 shows the results.

It was clarified that the anti-CD63 antibody/anti-miR nucleic acid conjugate inhibited exosome miR21-dependent cell proliferation.

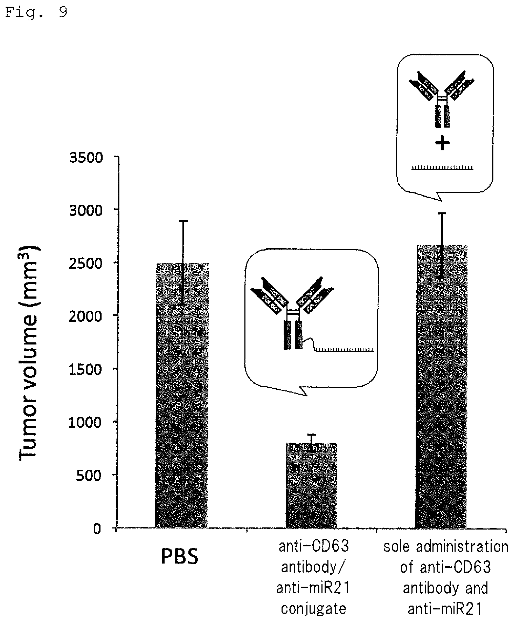

### Example 5: Function inhibiting effect of anti-CD63 antibody/anti-miR 21 nucleic acid conjugate in vivo (Fig. 9)

It was evaluated whether the anti-CD63 antibody/anti-miR nucleic acid conjugate exerts a microRNA function inhibiting effect also in vivo.

Cal27 (oral epithelial cancer cells) was transplanted to a gluteal region of nude mice (nu/nu BALB) in an amount of 500,000/200 µL/PBS. After 14 days, the tumor system was measured. A control (PBS) was prepared by administering only PBS together with Cal27; the control was compared with an anti-CD63 antibody/anti-miR21 nucleic acid conjugate (anti-CD63 IgG-9r + anti-miR21), or an anti-CD63 antibody + anti-miR21 administration group (sole administration in each group). Fig. 9 shows the results.

It was clarified that the conjugate of the present invention had a tumor proliferation inhibiting effect in vivo.

## Claims

1. A conjugate comprising an antibody or antibody fragment targeting an exosome surface antigen, and an inhibitor of a gene or an expression product thereof, wherein the antibody or antibody fragment and the inhibitor of a gene or an expression product thereof are covalently bonded either directly or via a linker, or are non-covalently bonded.

2. The conjugate according to claim 1, wherein the exosome surface antigen is CD9, CD63, CD81 or CD147.

3. The conjugate according to claim 1 or 2, wherein the antibody or antibody fragment is modified with a peptide containing at least one amino acid selected from the group consisting of cysteine, arginine, lysine and ornithine, and the inhibitor of a gene or an expression product thereof is bonded to the peptide via a covalent bond (S-S bond), a coordinate bond, or a non-covalent bond.

4. The conjugate according to claim 3, wherein the peptide further comprises glycine or alanine.

5. The conjugate according to claim 3, wherein the peptide is polylysine or polyarginine.

6. The conjugate according to claim 5, wherein the peptide is polyarginine.

7. The conjugate according to any one of claims 1 to 6, wherein the inhibitor of a gene or an expression product thereof is anti-miRNA nucleic acid, and the anti-miRNA nucleic acid is a nucleic acid that inhibits miRNA function by forming a complementary strand with miRNA contained in the exosome.

8. The conjugate according to claim 1, wherein the antibody or antibody fragment is anti-CD63 antibody.

9. The conjugate according to claim 1, wherein the inhibitor of a gene or an expression product thereof is an inhibitor of miRNA or a gene contained in the exosome.

10. The conjugate according to claim 1, wherein the inhibitor of a gene or an expression product thereof is an miRNA inhibitor.

11. The conjugate according to claim 1, wherein the antibody or antibody fragment is a monoclonal antibody, a single-chain antibody, Fab, Fab', F(ab')₂, Fv, or scFv.

12. A cancer proliferation and/or metastasis inhibitor, comprising the conjugate according to any one of claims 1 to 11.


SEQUENCE LISTING

<110>  Kyoto University
<110>  KYOTO PHARMACEUTICAL UNIVERSITY

<120>  Complex capable of inhibiting miRNA function in exosome, agent for
inhibiting of cancer metastasis and/or proliferation

<130>  P16-213WO

<150>  JP 2016-028924
<151>  2016-02-18

<160>  2

<170>  PatentIn version 3.5

<210>  1
<211>  29
<212>  RNA
<213>  Artificial Sequence

<220>
<223>  antisense RNA

<400>  1
agucaauagg gugugugaga gacuuacug                                         29

<210>  2
<211>  9
<212>  PRT
<213>  Artificial Sequence

<220>
<223>  Polyargininine

<400>  2
Arg Arg Arg Arg Arg Arg Arg Arg Arg                                     9
                1               5                   9